# FASCICULE DE BREVET EUROPEEN

(11) **EP 1 047 401 B1**
(45) Date de publication et mention de la délivrance du brevet: **26.03.2003**
(21) Numéro de dépôt: 99947529.6
(22) Date de dépôt: 11.10.1999
(51) Int. Cl.: A61K 7/50, A61K 7/06, A61K 7/48

(54) **COMPOSITIONS COSMETIQUES CONTENANT UN TENSIOACTIF ESTER D'ALKYLPOLYGLYCOSIDE ANIONIQUE ET UN AGENT CONDITIONNEUR LIQUIDE INSOLUBLE DANS L'EAU ET LEURS UTILISATIONS**
KOSMETISCHE ZUSAMMENSETZUNGEN ENTHALTEND EINEN ANIONISCHEN TENSID VOM TYP ALKYLPOLYGLYKOSIDESTER UND EIN FLÜSSIGES WASSERUNLÖSLICHES KONDITIONIERUNGSMITTEL SOWIE DEREN VERWENDUNGEN
COSMETIC COMPOSITIONS CONTAINING AN ANIONIC ALKYLPOLYGLYCOSIDE ESTER SURFACTANT AND A WATER INSOLUBLE LIQUID CONDITIONING AGENT AND THEIR USES

(30) Priorité: 12.11.1998 FR 9814215
(43) Date de publication de la demande: 02.11.2000
(73) Titulaire: L'OREAL, 75008 Paris (FR)
(72) Inventeur: CAUWET-MARTIN, Danièle, F-75011 Paris (FR); RESTLE, Serge, F-95390 Saint-Prix (FR)
(74) Mandataire: Dodin, Catherine
(86) Numéro de dépôt international: FR9902435
(87) Numéro de publication internationale: WO00028963

(56) Documents cités:
- EP-A- 0 510 564
- EP-A- 0 510 565
- EP-A- 0 699 472
- EP-A- 0 821 949
- WO-A-93/08204
- WO-A-94/27574
- DE-A- 19 617 019

## Description

La présente invention concerne de nouvelles compositions cosmétiques comprenant dans un milieu cosmétiquement acceptable au moins un tensioactif anionique de type ester d'alkylpolyglycoside carboxylique et au moins un agent conditionneur liquide à une température inférieure ou égale à 30 °C, non siliconé, non volatil et insoluble dans l'eau.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

Aussi, pour améliorer les propriétés cosmétiques des compositions cosmétiques et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétée, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes.

On a déjà proposé d'utiliser les huiles végétales ou animales en tant qu'agent conditionneur. Cependant, les compositions classiques ont des propriétés détergentes et moussantes non satisfaisantes. De plus les matières kératiniques traitées avec ces compositions présentent le plus souvent un toucher gras rédhibitoire.

WO9427571 et EP550276 décrivent des compositions détergentes contenant des tensioactifs anioniques du type alkylgalactoside uronate ne contenant pas de fonction ester.
L'article "New ultra mild naturally derived surfactants" (DCI mars 1997 p. 51) divulgue des compositions contenant un tensioactif anionique ester d'alkylpolyglycoside carboxylique et des huiles essentielles ou des huiles solubilisées.

L'invention a donc pour but de proposer des compositions cosmétiques présentant des propriétés cosmétiques améliorées, en particulier le démêlage et la douceur des cheveux.

Or, la demanderesse a maintenant trouvé qu'un tensioactif anionique de type ester d'alkylpolyglycoside carboxylique permettait d'atteindre ces buts.

Ces nouvelles compositions permettent de déposer une quantité plus importante d'huile sur les matières kératiniques (notamment les cheveux) qu'avec une composition classique, mais sans toucher ou aspect visuel gras.

Les compositions conformes à l'invention confèrent aux matières kératiniques notamment les cheveux, un remarquable effet traitant qui se manifeste notamment par une facilité de démêlage, ainsi qu'un apport de volume, de légèreté, de lissage, de douceur et de souplesse et de discipline sans aucune sensation de gras.

L'invention a ainsi pour objet une composition cosmétique, caractérisée en ce qu'elle comprend, dans un milieu cosmétiquement acceptable, au moins un agent conditionneur liquide à une température inférieure ou égale à 30 °C, non siliconé, non volatil et insoluble dans l'eau, au moins un tensioactif anionique de type ester d'alkylpolyglycoside carboxylique ou ses sels et un ou plusieurs polymères cationiques.

Un autre objet de l'invention concerne un procédé de traitement des matières kératiniques, telles que les cheveux, caractérisé en ce qu'il consiste à appliquer sur lesdites matières des compositions cosmétiques selon l'invention.

Selon la présente invention, par matières kératiniques, on comprend les cheveux, les cils, les sourcils, la peau, les ongles, les muqueuses ou le cuir chevelu et plus particulièrement les cheveux.

Les différents objets de l'invention vont maintenant être détaillés. L'ensemble des significations et définitions des composés utilisés dans la présente invention données ci-dessous sont valables pour l'ensemble des objets de l'invention.

Les tensioactifs anioniques de type ester d'alkylpolyglycoside carboxylique peuvent avoir la structure suivante :

R₁-(O-R₂)ₜO-(G)ᵥX (I)

dans laquelle R₁ représente un radical hydrocarboné linéaire ou ramifié, saturé ou insaturé en C₆-C₃₀, de préférence un radical alkyle linéaire ou ramifié, saturé ou insaturé, comportant environ de 8 à 24 atomes de carbone, un radical alkylphényle dont le radical alkyle linéaire ou ramifié comporte environ de 8 à 24 atomes de carbone, R₂ représente un radical alkylène comportant de 2 à 4 atomes de carbone, G représente un sucre réduit comportant de 5 à 6 atomes de carbone, t désigne une valeur allant de 0 à 10 et v désigne une valeur allant de 1 à 15.
X désigne un radical comprenant au moins une fonction acide carboxylique ou ses sels. X forme une liaison ester avec un hydroxyle du sucre de préférence en position 4 ou 6.
X peut être choisi parmi les radicaux suivants : 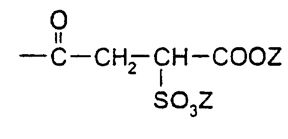 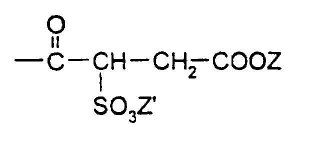 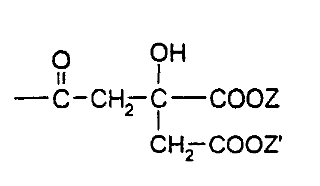 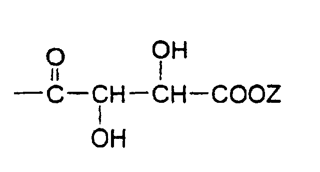 Z et Z', identiques ou différents, désignent un atome d'hydrogène, un cation minéral ou organique tel que :
un métal alcalin (par exemple Na⁺, K⁺), NH₄⁺, les ammoniums issus des aminoacides basiques tels que la lysine, l'arginine, la sarcosine, l'ornithine, la citrulline ou bien encore des amino-alcools tels que la monoéthanolamine, la diéthanolamine, la triéthanolamine, la glucamine, la N-méthyl glucamine, l'amino-3 propanediol-1,2.

Des esters d'alkylpolyglycosides préférés selon la présente invention sont des composés de formule (I) dans laquelle R₁ désigne plus particulièrement un radical alkyle saturé ou insaturé, linéaire ou ramifié comportant de 8 à 18 atomes de carbone, t désigne une valeur allant de 0 à 3 et plus particulièrement encore égale à 0, G peut désigner le glucose, le fructose ou le galactose, de préférence le glucose. Le degré de polymérisation du saccharide, i.e. la valeur de v dans la formule (I), peut aller de 1 à 15. Selon l'invention, on préfère les sucres réduits contenant 80%, ou plus, de sucres dont le degré de polymérisation (S) prend une valeur allant de 1 à 4. Le degré moyen de polymérisation est plus particulièrement compris entre 1 et 2.
Les liaisons glycosidiques sont de type 1-6 ou 1-4 et de préférence 1-4.

Encore plus particulièrement, R1 désigne un radical oléyle ou un mélange de radicaux en C₈-C₁₈ dérivés du suif ou de coprah et t=0.

Ces tensioactifs sont notamment décrits dans les demandes de brevet EP510564 et EP510565.

Parmi les tensioactifs de formule (I), on peut citer les produits commercialisés sous la dénomination EUCAROL® AEG-SS, EUCAROL® AEG-EC et EUCAROL® AEG-ET par la société CESALPINA.

Selon l'invention, le tensioactif anionique de type ester d'alkylpolyglycoside carboxylique peut représenter de 0,5 % à 30 % en poids, de préférence de 1 % à 25 % en poids, et encore plus préférentiellement de 2,5 % à 15 % en poids par rapport au poids total de la composition finale.

Selon l'invention, les agents conditionneurs liquides à une température inférieure ou égale à 30 °C, non siliconés, non volatils et insolubles dans l'eau peuvent être choisis parmi les poly-α-oléfines, les huiles fluorées, les esters d'acides carboxyliques, les huiles minérales, végétales ou animales, les alcools gras et leurs mélanges. Les agents conditionneurs préférés selon l'invention sont les poly-α-oléfines, les esters d'acides carboxyliques ayant au moins 10 atomes de carbone et les huiles végétales.

Les agents conditionneurs liquides insolubles dans l'eau sont insolubles dans l'eau à une concentration supérieure ou égale à 0,1% en poids dans l'eau à 25°C, c'est à dire qu'ils ne forment pas une solution isotrope transparente.

Un agent conditionneur non volatil, selon l'invention, est un agent conditionneur qui présente une température d'ébullition supérieure à 240°C sous 760 mm de Hg.

Les poly-α-oléfines sont en particulier :
- de type polybutène, hydrogéné ou non, et de préférence polyisobutène, hydrogéné ou non.
   On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.
   A titre d'exemples de poly-α-oléfines utilisables dans le cadre de la présente invention, on peut plus particulièrement mentionner les produits vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.
   De tels produits sont vendus par exemple sous les dénominations ETHYLFLO par la société ETHYL CORP., et d'ARLAMOL PAO par la société ICI.

Le nombre total de carbone des esters est généralement supérieur ou égal à 10 et de préférence inférieure à 100 et plus particulièrement inférieur à 80. De préférence, ils ne possèdent pas de propriétés tensioactives.

Les esters d'acides peuvent être mono, di, tri ou tétracarboxyliques.

Les esters d'acides monocarboxyliques sont notamment les monoesters d'acides aliphatiques saturés ou insaturés, linéaires ou ramifiés en C₁-C₂₆ et d'alcools aliphatiques saturés ou insaturés, linéaires ou ramifiés en C₁-C₂₆ , le nombre total de carbone des esters étant supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle : l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le myristate d'isopropyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octytdodécyle ; le myristate d'octyldodécyle ; l'érucate d'oléyle : les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle.

On peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.
On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle.

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

Les huiles fluorées sont par exemple les perfluoropolyéthers décrits notamment dans la demande de brevet EP-A-486135 et les composés fluorohydrocarbonées décrites notamment dans la demande de brevet WO 93/11103.

Le terme de composés fluorohydrocarbonés désigne des composés dont la structure chimique comporte un squelette carboné dont certains atomes d'hydrogène ont été substitués par des atomes de fluor.

Les huiles fluorées peuvent également être des fluorocarbures tels que des fluoramines par exemple la perfluorotributylamine, des hydrocarbures fluorés, par exemple le perfluorodécahydronaphtalène, des fluoroesters et des fluoroethers ;

Les perfluoropolyéthers sont par exemple vendus sous les dénominations commerciales FOMBLIN par la société MONTEFLUOS et KRYTOX par la société DU PONT.

Parmi les composés fluorohydrocarbonés, on peut également citer les esters d'acides gras fluorés tels que le produits vendu sous la dénomination NOFABLE FO par la société NIPPON OIL.

Les huiles pouvant être utilisées dans les compositions de l'invention sont choisies préférentiellement dans le groupe formé par :
- les huiles animales ou végétales formées par des esters d'acide gras et de polyols, en particulier les triglycérides liquides, par exemple les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone en particulier alkyle ou alkényle, par exemple, l'huile de Purcellin ou la cire liquide de jojoba ;

Les alcools gras liquides peuvent être choisis parmi les alcools gras en C₈-C₂₂, linéaires ou ramifiés ; ils sont éventuellement oxyalkylénés avec 1 à 15 moles d'oxyde d'alkylène ou polyglycérolés avec 1 à 6 moles de glycérol.

Plus particulièrement, les alcools gras liquides sont choisis parmi les alcools isostéarylique, isocétylique et oléique, les alcools lauriques oxyéthylénés de 2 à 8 moles d'oxyde d'éthytène et leurs mélanges.

Selon l'invention, l'agent conditionneur liquide, non siliconé, non volatil et insoluble dans l'eau peut représenter de 0,001 % à 10 % en poids, de préférence de 0,005 % à 5 % en poids, et encore plus préférentiellement de 0,01 % à 3 % en poids, par rapport au poids total de la composition.

Les compositions de l'invention contiennent en outre avantageusement au moins un autre agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, cationiques ou leurs mélanges.

Les tensioactifs additionnels convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention. de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propyfène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras potyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères additionnels, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₅) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide carboxylique présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide carboxylique présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particuiièrement préféré est un mélange constitué d'au moins un agent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;
- soit un agent tensioactif amphotère tel que les alkylbétaïnes en particulier la cocobétaïne commercialisée sous la dénomination "DEHYTON® AB 30" en solution aqueuse à 32 % de MA par la société HENKEL ou tel que les alkyl (C₈-C₂₀) amidoalkyl (C₁-C₆) bétaïnes en particulier la TEGOBETAINE® F 50 commercialisée par la société GOLDSCHMIDT.

On peut également utiliser des tensioactifs cationiques parmi lesquels on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

La composition de l'invention peut également contenir au moins un additif choisi parmi les épaississants, les parfums, les agents nacrants, les conservateurs, les filtres solaires, les agents tensio-actifs cationiques, les polymères anioniques ou non ioniques ou cationiques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les silicones et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 50% en poids par rapport au poids total de la composition. La quantité précise de chaque additif est déterminée facilement par l'homme du métier selon sa nature et sa fonction.

Le milieu cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.
De préférence , la composition comprend de 50 à 95 % en poids d'eau par rapport au poids total de la composition.

Les compositions détergentes selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions selon l'invention comprennent un ou plusieurs polymères cationiques.
Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

Les compositions selon l'invention peuvent contenir également des synergistes de mousses tels que des 1,2-alcanediols en C₁₀-C₁₈ ou des alcanolamides gras dérivés de mono ou de diéthanolamine.

Les compositions conformes à l'invention peuvent être utilisées pour le lavage ou le traitement des matières kératiniques telles que les cheveux, la peau, les cils, les sourcils, les ongles, les lèvres, le cuir chevelu et plus particulièrement les cheveux.

En particulier, les compositions selon l'invention sont des compositions détergentes telles que des shampooings, des gels-douche et des bains moussants.
Dans ce mode de réalisation de l'invention, les compositions comprennent une base lavante, généralement aqueuse. La base lavante peut comprendre uniquement le tensioactif anionique de type ester d'alkylpolyglycoside carboxylique ou comprendre d'autres tensioactifs additionnels.

Le ou les tensioactifs additionnels peuvent être indifféremment choisis, seuls ou en mélanges, au sein des tensioactifs anioniques, amphotères, non ioniques et cationiques tels que définis ci-dessus.

La quantité et la qualité de la base lavante sont celles suffisantes pour conférer à la composition finale un pouvoir moussant et/ou détergent satisfaisant.

Ces compositions détergentes sont de préférence moussantes et le pouvoir moussant des compositions selon l'invention, caractérisé par une hauteur de mousse, est généralement supérieur à 75 mm ; de préférence, supérieur à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Ainsi, selon l'invention, la base lavante peut représenter de 4 % à 50 % en poids, de préférence de 6 % à 35 % en poids, et encore plus préférentieilement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin, le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques, de gel, de lait, de crème, d'émulsion, de lotion épaissie ou de mousse et être utilisées pour la peau, les ongles, les cils, les lèvres et plus particulièrement les cheveux.

L'invention va être maintenant plus complètement illustrée à l'aide des exemples suivants qui ne sauraient être considérés comme la limitant aux modes de réalisation décrits.

Dans tout ce qui suit ou ce qui précède, les pourcentages exprimés sont en poids. Dans les exemples, MA signifie matière active.

### EXEMPLE 1

On a réalisé une composition de shampooing conforme à l'invention :

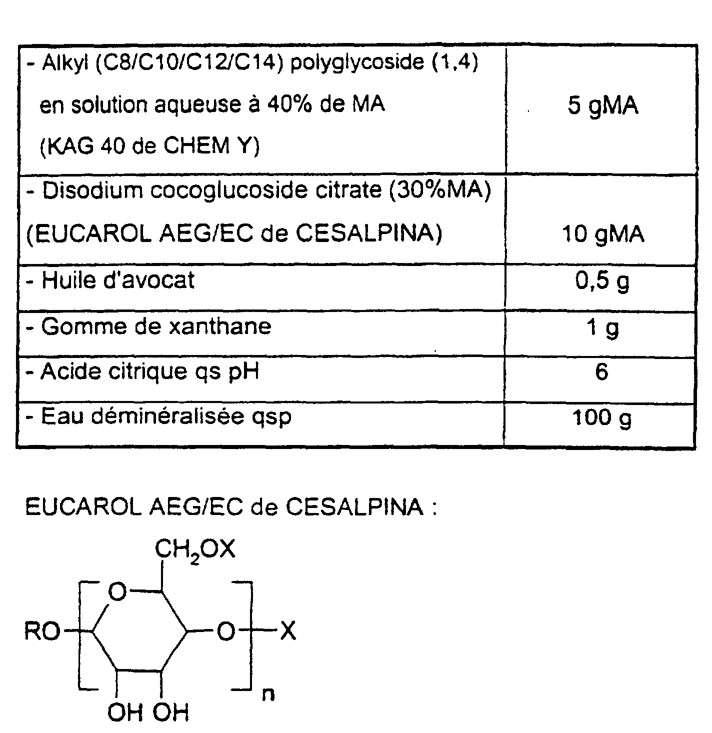

Un des radicaux X désigne hydrogène et l'autre : 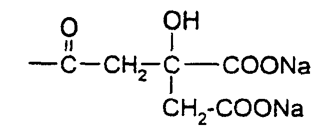 R est un radical dérivé d'alcool de coprah

On effectue un shampooing en appliquant environ 12 g de la composition A sur des cheveux naturels préalablement mouillés. On fait mousser le shampooing puis on rince abondamment à l'eau.

Les cheveux sont gainés et corporisés.

### EXEMPLE 2

On a réalisé une composition de shampooing conforme à l'invention :

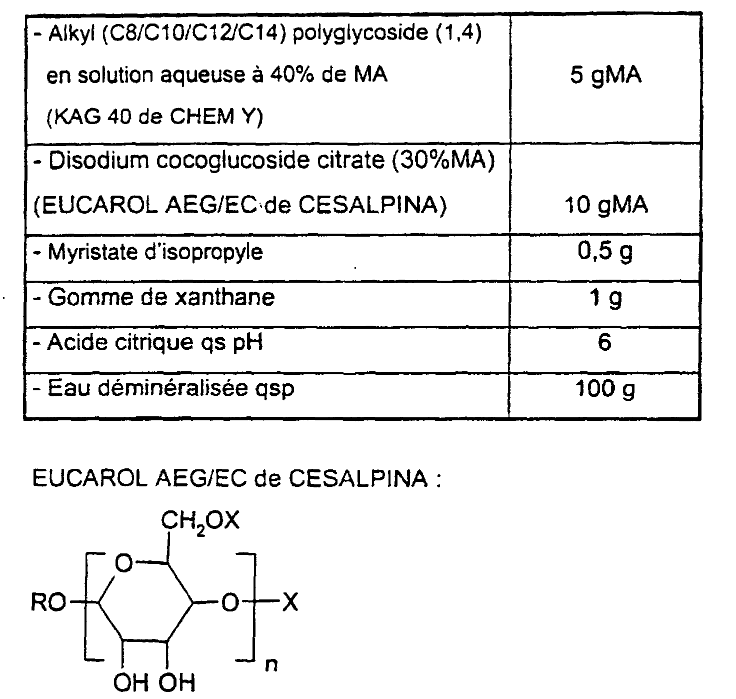

Un des radicaux X désigne hydrogène et l'autre : 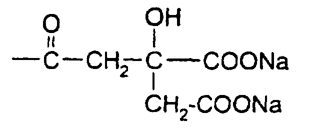 R est un radical dérivé d'alcool de coprah

On effectue un shampooing en appliquant environ 12 g de la composition A sur des cheveux naturels préalablement mouillés. On fait mousser le shampooing puis on rince abondamment à l'eau.

Les cheveux sont gainés et corporisés.

### EXEMPLE 3

On a réalisé une composition de shampooing conforme à l'invention :

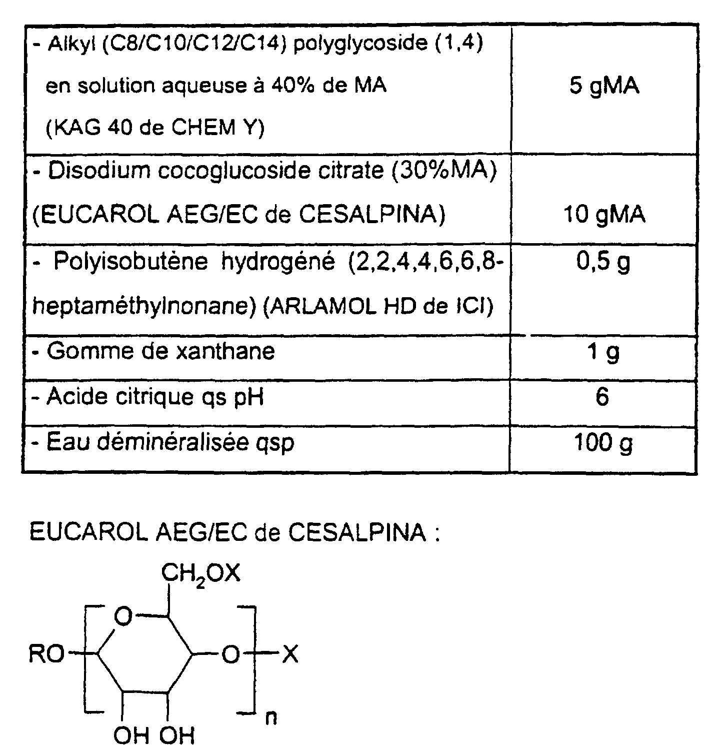

Un des radicaux X désigne hydrogène et l'autre : 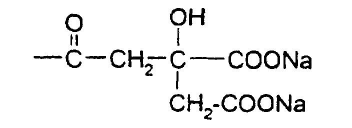 R est un radical dérivé d'alcool de coprah

On effectue un shampooing en appliquant environ 12 g de la composition A sur des cheveux naturels préalablement mouillés. On fait mousser le shampooing puis on rince abondamment à l'eau.

Les cheveux sont gainés et corporisés.

## Revendications

1. Composition cosmétique, **caractérisée par le fait qu'**elle comprend, dans un milieu cosmétiquement acceptable, au moins un agent conditionneur liquide à une température inférieure ou égale à 30 °C, non siliconé, non volatil et insoluble dans l'eau, au moins un tensioactif anionique de type ester d'alkylpolyglycoside carboxylique ou ses sels et un ou plusieurs polymères cationiques.

2. Composition selon la revendication 1, **caractérisée par le fait que** ledit tensioactif anionique de type ester d'alkylpolyglycoside carboxylique présente la formule (I) :
R₁-(O-R₂)ₜO-(G)ᵥX (I)
dans laquelle R₁ représente un radical hydrocarboné linéaire ou ramifié, saturé ou insaturé en C₆-C₃₀ , R₂ représente un radical alkylène comportant de 2 à 4 atomes de carbone, G représente un sucre réduit comportant de 5 à 6 atomes de carbone, t désigne une valeur allant de 0 à 10 et v désigne une valeur allant de 1 à 15, X désigne un radical comprenant au moins une fonction acide carboxylique ou ses sels et X forme une liaison ester avec un hydroxyle du sucre.

3. Composition selon la revendication 2, **caractérisée par le fait que** le radical X est choisi parmi les radicaux suivants : 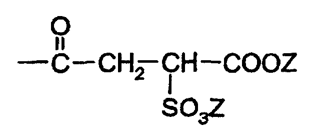 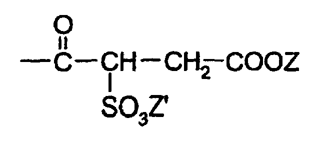 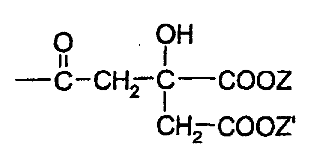 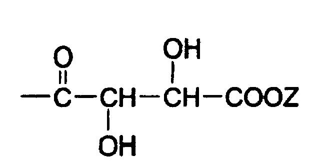 Z et Z', identiques ou différents, désignent un atome d'hydrogène, un cation minéral ou organique.

4. Composition selon l'une quelconque des revendications 2 ou 3, **caractérisée par le fait que** le radical R1 désigne un radical alkyle linéaire ou ramifié, saturé ou insaturé, comportant de 8 à 24 atomes de carbone, un radical alkylphényle dont le radical alkyle linéaire ou ramifié comporte de 8 à 24 atomes de carbone.

5. Composition selon l'une quelconque des revendications 2 à 4, **caractérisée par le fait que** le radical R1 est un radical oléyle ou un radical dérivé du coprah.

6. Composition selon l'une quelconque des revendications 2 à 5, **caractérisée par le fait que** t désigne une valeur allant de 0 à 3 et plus particulièrement encore égale à 0.

7. Composition selon l'une quelconque des revendications 2 à 6, **caractérisée par le fait que** G désigne le glucose, le fructose ou le galactose, de préférence le glucose.

8. Composition selon l'une quelconque des revendications 2 à 7, **caractérisée par le fait que** la valeur de v va de 1 à 15 et de préférence de 1 à 4.

9. Composition selon l'une quelconque des revendications 1 à 8, **caractérisée par le fait que** lesdits agents conditionneurs sont choisis les poly-α-oléfines, les huiles fluorées, les esters d'acides carboxyliques, les huiles minérales, végétales ou animales, les alcools gras, et leurs mélanges.

10. Composition selon la revendication 9, **caractérisée par le fait que** lesdites poly-α-oléfines sont :
- de type polybutène, hydrogéné ou non, et de préférence polyisobutène, hydrogéné ou non.
- de type polydécène, hydrogéné ou non.

11. Composition selon la revendication 9, **caractérisée par le fait que** lesdits esters d'acide carboxylique sont choisis parmi le palmitate d'éthyle, le palmitate d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle, l'isononanate d'isononyle et l'octanoate de cétyle.

12. Composition selon l'une quelconque des revendications 1 à 11, **caractérisée en ce que** le tensioactif anionique de type ester d'alkylpolyglycoside carboxylique est présent dans les compositions à une concentration comprise entre 0,5 et 30 % en poids, de préférence entre 1 et 25% en poids et plus particulièrement entre 2,5 et 15 % en poids par rapport au poids total de la composition.

13. Composition selon l'une quelconque des revendications 1 à 12, **caractérisée en ce que** ledit agent conditionneur est présent à une concentration comprise entre 0,001 % et 10 % en poids par rapport au poids total de la composition, de préférence entre 0,005 % et 5 % en poids et en particulier entre 0,01 % et 3 % en poids.

14. Composition selon l'une quelconque des revendications 1 à 13, **caractérisée par le fait qu'**elle comprend en outre au moins un agent tensioactif additionnel choisi parmi les tensioactifs anioniques, cationiques, non ioniques, amphotères et leurs mélanges.

15. Compositions selon la revendication 14, **caractérisées par le fait que** le ou les agents tensioactifs additionnels sont présents à une concentration comprise entre 0,1% et 60% en poids, de préférence entre 3% et 40% en poids, et encore plus préférentiellement entre 5% et 30% en poids, par rapport au poids total de la composition.

16. Composition selon l'une quelconque des revendications 1 à 15, **caractérisée par le fait qu'**elle comprend en outre au moins un additif choisi parmi les épaississants, les parfums, les agents nacrants, les conservateurs, les filtres solaires, les agents tensioactifs cationiques, les polymères anioniques ou non ioniques ou cationiques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les silicones .

17. Composition selon l'une quelconque des revendications 1 à 16, **caractérisées par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour la peau.

18. Utilisation d'une composition telle que définie dans l'une quelconque des revendications précédentes pour le lavage des matières kératiniques telles que les cheveux.

19. Procédé de traitement des matières kératiniques, telles que les cheveux, **caractérisé en ce qu'**il consiste à appliquer sur lesdites matières une composition cosmétique selon l'une des revendications 1 à 17, puis à effectuer éventuellement un rinçage à l'eau.

## Patentansprüche

1. Kosmetische Zusammensetzung, **dadurch gekennzeichnet, dass** sie in einem kosmetisch akzeptablen Medium mindestens ein bei einer Temperatur von 30 °C oder darunter flüssiges, nicht siliconhaltiges, nicht flüchtiges und in Wasser unlösliches Konditioniermittel, mindestens einen anionischen grenzflächenaktiven Stoff vom Typ der Alkylpolyglycosid-carbonsäureester und ihrer Salze und ein oder mehrere kationische Polymere enthält.

2. Zusammensetzung nach Anspruch 1, **dadurch gekennzeichnet, dass** der anionische grenzflächenaktive Stoff vom Typ der Alkylpolyglycosid-carbonsäureester der folgenden Formel (I) entspricht:
R₁-(O-R₂)ₜO-(G)ᵥX (I)
wobei die Gruppe R₁ eine geradkettige oder verzweigte, gesättigte oder ungesättigte Kohlenwasserstoffgruppe mit 6 bis 30 Kohlenstoffatomen bedeutet, die Gruppe R₂ eine Alkylengruppe mit 2 bis 4 Kohlenstoffatomen ist, die Gruppe G einen reduzierten Zucker mit 5 bis 6 Kohlenstoffatomen bedeutet, t einen Wert im Bereich von 0 bis 10 und v einen Wert im Bereich von 1 bis 15 bezeichnet und X eine Gruppe ist, die mindestens eine Carbonsäuregruppe oder ihre Salze enthält, wobei X mit einer Hydroxygruppe des Zuckers eine Esterbindung ausbildet.

3. Zusammensetzung nach Anspruch 2, **dadurch gekennzeichnet, dass** die Gruppe X unter den folgenden Gruppen ausgewählt ist: 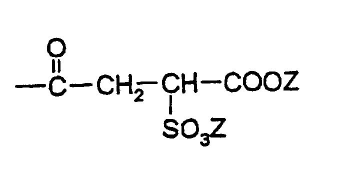 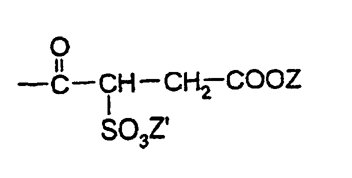 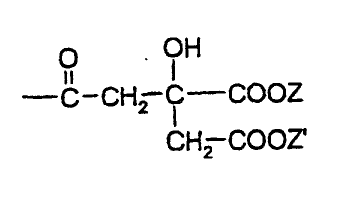 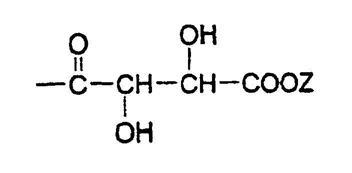 wobei Z und Z', die identisch oder voneinander verschieden sind, Wasserstoff oder ein organisches oder anorganisches Kation bedeuten.

4. Zusammensetzung nach einem der Ansprüche 2 oder 3, **dadurch gekennzeichnet, dass** die Gruppe R₁ eine geradkettige oder verzweigte, gesättigte oder ungesättigte Alkylgruppe mit 8 bis 24 Kohlenstoffatomen oder eine Alkylphenylgruppe bedeutet, deren geradkettige oder verzweigte Alkylgruppe 8 bis 24 Kohlenstoffatome. aufweist.

5. Zusammensetzung nach einem der Ansprüche 2 bis 4, **dadurch gekennzeichnet, dass** die Gruppe R₁ die Oleylgruppe oder eine von Kopra abgeleitete Gruppe ist.

6. Zusammensetzung nach einem der Ansprüche 2 bis 5, **dadurch gekennzeichnet, dass** t eine Zahl von 0 bis 3 und insbesondere 0 bedeutet.

7. Zusammensetzung nach einem der Ansprüche 2 bis 6, **dadurch gekennzeichnet, dass** G Glucose, Fructose oder Galactose und vorzugsweise Glucose bedeutet.

8. Zusammensetzung nach einem der Ansprüche 2 bis 7, **dadurch gekennzeichnet, dass** v im Bereich von 1 bis 15 und vorzugsweise 1 bis 4 liegt.

9. Zusammensetzung nach einem der Ansprüche 1 bis 8, **dadurch gekennzeichnet, dass** die Konditioniermittel unter den Poly-α-olefinen, fluorierten Ölen, Carbonsäureestern, Mineralölen, pflanzlichen Ölen, tierischen Ölen, Fettalkoholen und deren Gemischen ausgewählt sind.

10. Zusammensetzung nach Anspruch 9, **dadurch gekennzeichnet, dass** die Poly-α-olefine sind:
- vom Polybutentyp, wobei die Verbindungen hydriert oder nicht hydriert sind, vorzugsweise gegebenenfalls hydriertes Polyisobuten;
- vom Polydecentyp, wobei die Verbindungen hydriert oder nicht hydriert sind.

11. Zusammensetzung nach Anspruch 9, **dadurch gekennzeichnet, dass** die Carbonsäureester unter Ethylpalmitat, Isopropylpalmitat, 2-Ethylhexylpalmitat, 2-Octyldecylpalmitat, Alkylmyristaten, wie Isopropylmyristat, Butylmyristat, Cetylmyristat und 2-Octyldodecylmyristat, Hexylstearat, Butylstearat, Isobutylstearat, Dioctylmalat, Hexyllaurat, 2-Hexyldecyllaurat, Isononylisononanat und Cetyloctanoat ausgewählt sind.

12. Zusammensetzung nach einem der Ansprüche 1 bis 11, **dadurch gekennzeichnet, dass** der anionische grenzflächenaktive Stoff vom Typ Alkylpolyglycosid-carbonsäureester in den Zusammensetzungen in einer Konzentration von 0,5 bis 30 Gew.-%, vorzugsweise 1 bis 25 Gew.-% und insbesondere 2,5 bis 15 Gew.-%, bezogen auf das Gesamtgewicht der Zusammensetzung, vorliegt.

13. Zusammensetzung nach einem der Ansprüche 1 bis 12, **dadurch gekennzeichnet, dass** das Konditioniermittel in einer Konzentration von 0,001 bis 10 Gew.-%, bezogen auf das Gesamtgewicht der Zusammensetzung, vorzugsweise 0,005 bis 5 Gew.-% und insbesondere 0,01 bis 3 Gew.-%, bezogen auf das Gesamtgewicht der Zusammensetzung, vorliegt.

14. Zusammensetzung nach einem der Ansprüche 1 bis 13, **dadurch gekennzeichnet, dass** sie ferner mindestens einen zusätzlichen grenzflächenaktiven Stoff enthält, der unter den anionischen, kationischen, nichtionischen oder amphoteren grenzflächenaktiven Stoffen und deren Gemischen ausgewählt ist.

15. Zusammensetzung nach Anspruch 14, **dadurch gekennzeichnet, dass** der oder die zusätzlichen grenzflächenaktiven Stoffe in einer Konzentration von 0,1 bis 60 Gew.-%, vorzugsweise 3 bis 40 Gew.-% und noch bevorzugter 5 bis 30 Gew.-%, bezogen auf das Gesamtgewicht der Zusammensetzung, vorliegen.

16. Zusammensetzung nach einem der Ansprüche 1 bis 14, **dadurch gekennzeichnet, dass** sie ferner mindestens einen Zusatzstoff enthält, der unter den Verdickungsmitteln, Parfums, Perlglanzpigmenten, Konservierungsmitteln, Sonnenschutzfiltern, kationischen grenzflächenaktiven Stoffen, anionischen, nichtionischen, katiönischen oder amphoteren Polymeren, Proteinen, Proteinhydrolysaten, Ceramiden, Pseudoceramiden, Fettsäuren mit geraden oder verzweigten C₁₆₋₄₀-Ketten, beispielsweise 18-Methyleicosansäure, Hydroxysäuren, Vitaminen, Panthenol und Siliconen ausgewählt ist.

17. Zusammensetzung nach einem der Ansprüche 1 bis 16, **dadurch gekennzeichnet, dass** sie als Haarwaschmittel, Haarpflegemittel, Zusammensetzung für eine Dauerwelle, Entkräuselung, Färbung oder Entfärbung der Haare, Zusammensetzung, die ausgespült wird und zwischen den beiden Schritten einer Dauerwelle oder Entkräuselung aufgetragen wird, oder reinige Zusammensetzung für die Haut vorliegt.

18. Verwendung einer Zusammensetzung nach einem der vorhergehenden Ansprüche zum Waschen von Keratinsubstanzen, wie dem Haar.

19. Verfahren zur Behandlung von Keratinsubstanzen, wie dem Haar, **dadurch gekennzeichnet, dass** auf die Keratinsubstanzen eine kosmetische Zusammensetzung nach einem der Ansprüche 1 bis 17 aufgetragen und gegebenenfalls anschließend mit Wasser gespült wird.

## Claims

1. Cosmetic composition, **characterized in that** it comprises, in a cosmetically acceptable medium, at least one nonsilicone, nonvolatile and water-insoluble conditioning agent liquid at a temperature less than or equal to 30°C, at least one anionic surfactant of the alkylpolyglycoside carboxylic ester type or its salts and one or more cationic polymers.

2. Composition according to Claim 1, **characterized in that** the said anionic surfactant of the alkylpolyglycoside carboxylic ester type has the formula (I):
R₁-(O-R₂)ₜO-(G)ᵥX (I)
in which R₁ represents a saturated or unsaturated, linear or branched C₆-C₃₀ hydrocarbon radical, R₂ represents an alkylene radical comprising from 2 to 4 carbon atoms, G represents a reduced sugar comprising 5 or 6 carbon atoms, t denotes a value ranging from 0 to 10 and v denotes a value ranging from 1 to 15, X denotes a radical comprising at least one carboxylic acid function or its salts and X forms an ester bond with a hydroxyl of the sugar.

3. Composition according to Claim 2, **characterized in that** the X radical is chosen from the following radicals: 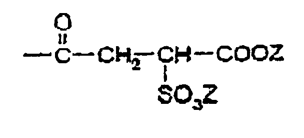 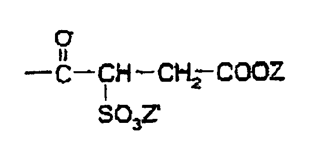 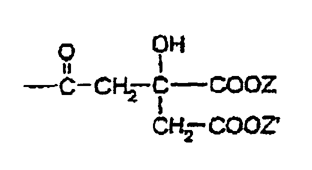 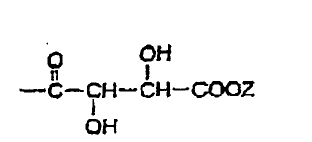 Z and Z', which are identical or different, denote a hydrogen atom, or an inorganic or organic cation.

4. Composition according to either of Claims 2 and 3, **characterized in that** the R₁ radical denotes a saturated or unsaturated, linear or branched alkyl radical comprising from 8 to 24 carbon atoms, an alkylphenyl radical in which the linear or branched alkyl radical comprises from 8 to 24 carbon atoms.

5. Composition according to any one of Claims 2 to 4, **characterized in that** the R₁ radical is an oleyl radical or a radical derived from copra.

6. Composition according to any one of Claims 2 to 5, **characterized in that** t demotes a value ranging from 0 to 3 and still more particularly equal to 0.

7. Composition according to any one of Claims 2 to 6, **characterized in that** G denotes glucose, fructose or galactose, preferably glucose.

8. Composition according to any one of Claims 2 to 7, **characterized in that** the value of v ranges from 1 to 15 and preferably from 1 to 4.

9. Composition according to any one of Claims 1 to 8, **characterized in that** the said conditioning agents are chosen from poly-α-olefins, fluorinated oils, esters of carboxylic acids, mineral, vegetable or animal oils, fatty alcohols and mixtures thereof.

10. Composition according to Claim 9, **characterized in that** the said poly-α-olefins are:
- of the polybutene type, hydrogenated or otherwise, and preferably polyisobutene type, hydrogenated or otherwise,
- of the polydecene type, hydrogenated or otherwise.

11. Composition according to Claim 9, **characterized in that** the said esters of a carboxylic acid are chosen from ethyl palmitate, isopropyl palmitate, 2-ethylhexyl palmitate, 2-octyldecyl palmitate, alkyl myristates such as isopropyl, butyl, cetyl or 2-octyldodecyl myristate, hexyl stearate, butyl stearate, isobutyl stearate; dioctyl malate, hexyl laurate, 2-hexyldecyl laurate, isononyl isononanate and cetyl octanoate.

12. Composition according to any one of Claims 1 to 11, **characterized in that** the anionic surfactant of the alkylpolyglycoside carboxylic ester type is present in the compositions at a concentration, of between 0.5 and 30% by weight, preferably between 1 and 25% by weight and more particularly between 2.5 and 15% by weight relative to the total weight of the composition.

13. Composition according to any one of Claims 1 to 12, **characterized in that** the said conditioning agent is present at a concentration of between 0.001% and 10% by weight relative to the total weight of the composition, preferably between 0.005% and 5% by weight and in particular between 0.01% and 3% by weight.

14. Composition according to any one of Claims 1 to 13, **characterized in that** it comprises, in addition, at least one additional surfactant chosen from anionic, cationic, nonionic and amphoteric surfactants and mixtures thereof.

15. Composition according to Claim 14, **characterized in that** the additional surfactant(s) are present at a concentration of between 0.1% and 60% by weight, preferably between 3% and 40% by weight and still more preferably between 5% and 30% by weight, relative to the total weight of the composition.

16. Composition according to any one of Claims 1 to 15, **characterised in that** it comprises, in addition, at least one additive chosen from thickeners, perfumes, pearlescent agents, preservatives, sunscreens, cationic surfactants, anionic or nonionic or cationic or amphoteric polymers, proteins, protein hydrolysates, ceramides, pseudoceramides, linear or branched C₁₆-C₄₀ chain fatty acids such as 18-methyleicosanoic acid, bydroxy acids, vitamins, panthenol and silicones.

17. Composition according to any one of Claims 1 to 16, **characterized in that** it is provided in the form of a shampoo, a hair conditioner, a composition. for permanent waving, straightening, dyeing or bleaching the hair, a rinse composition to be applied between the two stages of a permanent waving or hair straightening treatment, or a cleansing composition for the skin.

18. Use of a composition as defined in any one of the preceding claims for washing keratinous materials such as the hair.

19. Method of treating keratinous materials, such as the hair, **characterized in that** it consists in applying to the said materials a cosmetic composition according to one of Claims 1 to 17, and then in optionally carrying out a rinsing with water.
